# EUROPEAN PATENT APPLICATION

(11) **EP 2 177 211 A1**
(43) Date of publication of application: **21.04.2010**
(21) Application number: 08805395.4
(22) Date of filing: 30.07.2008
(51) Int. Cl.: A61K 9/127

(54) **ORGANIC-INORGANIC HYBRID MATERIAL FOR STORAGE AND RELEASE OF ACTIVE PRINCIPLES**

(30) Priority: 01.08.2007 ES 200702163
(71) Applicant: Consejo Superior De Investigaciones Científicas, 28006 Madrid (ES); Universidad Politecnia de Valencia, 46022 Valencia (ES)
(72) Inventor: CORMA CANOS, Avelino, E-46022 Valencia (ES); ARRICA, María Antonia, E-46022 Valencia (ES); DÍAZ MORALES, Urbano Manuel, E-46022 Valencia (ES)
(74) Representative: Pons Ariño, Angel
(86) International application number: PCT/ES2008/070150
(87) International publication number: WO 2009/024635

(57) **Abstract**

This invention relates to a hybrid material composed of organic-inorganic particles, **characterised in that** said particles have a diameter of between 10 and 800 nm, and it is structured in two portions:
- an inner portion that comprises a micellar phase wherein one or more active principles are immersed,
- an outer portion composed of an organic-inorganic network formed by inorganic units and organic units covalently bound to one another, forming a spherical network that coats the micellar phase, and to the use thereof in the storage and release of active principles.

## Description

### TECHNICAL FIELD OF THE INVENTION

This invention belongs to the field of structured organic-inorganic materials, particularly hybrid materials composed of nanoparticles that lodge molecules inside them, and, more particularly, materials that are useful to contain and release molecules with biomedical applications, amongst others.

### OBJECTS OF THE INVENTION

The first object of this invention is to generate a new type of particles of nanometric size the interior whereof is composed of a micellar phase that contains one or more stabilised active principles, and, organised around said principle or principles, there is an organic-inorganic coating susceptible to being modified to make the molecules lodged therein to exit.

A second object of the invention is a method of preparing the hybrid nanoparticles and the most suitable synthesis conditions to obtain a suitable product to be used for the storage and release of molecules and molecular structures.

### PRIOR STATE OF THE ART

In recent decades, one of the great objectives within the field of biocompatible materials has been the preparation of systems capable of storing and administering active principles in a controlled manner. In the beginning, due to their versatility, organic systems were the most widely used. These systems include micellae, liposomes and polymeric nanoparticles, and liposomal phases provided the best results for drug encapsulation [T. Nii, F. Ishii, International Journal of Pharmaceutics 298 (2005) 198-205*].* Specifically, liposomes are vesicles consisting of one or more concentric spheres of lipid layers separated from one another by water molecules. Their special composition makes them highly effective to encapsulate active principles, both hydrophilic and lipophilic, inside them, by interaction with the aqueous or phospholipid phase that makes them up. However, liposomes and, in general, all organic encapsulation systems have serious limitations, since they are unstable from a hydrothermal and a chemical standpoint; furthermore, they are quickly attacked and eliminated by the immunological system. All this makes it necessary to find new alternatives to resolve said disadvantages [S. Bégu, A.A. Pouëssel, D.A. Lerner, C. Tourné-Péteilh, J.M. Devoisselle, Journal of Controlled Release 118 (2007) 1-6]*.*

In other works, silicon particles are used, the preparation whereof has been widely described in the state of the art. This type of nanoparticles are stable toward external agents and, moreover, biocompatible, and bioactive molecules may be stored inside them during the synthesis thereof [N.E. Botterhuis, Q. Sun, P.C.M.M. Magusin, R.A. van Santen, N.A.J.M. Sommerdijk, Chemistry European Journal 12 (2006) 1448-1456]. These siliceous particles loaded with active principles may be obtained by combining different preparation methods, such as hydrolysis and condensation, spray-drying or emulsion methods. However, the most commonly used method is sol-gel technology, which is a simple inorganic polymerisation technique at ambient temperature, starting from neutral siliceous precursors [R.K. Rana, Y. Mastai, A. Gedanken, Advanced Materials 14 (2002) 1414-1418]. However, although the methodologies used allow for a precise control of the size of the siliceous particles obtained, they pose various problems in regards to the release of the internal active principle, since, depending on the porosity of the siliceous matrix and the size of the active molecule, said drug will be released with greater or lesser ease.

In order to overcome this disadvantage, siliceous nanoparticles were prepared with mesoporous outer walls containing active principles inside them, using surfactants during the preparation process [H. Djojoputro, X.F. Zhou, S.Z. Qiao, L.Z. Wang, C.Z. Yu, G.Q. Lu, Journal of the American Chemical Society 128 (2006) 6320-6321*].* The purpose of this methodology was to facilitate the diffusion of the bioactive molecules toward the exterior of the nanosphere, without any restrictions being imposed by the presence of pores with a very limited diameter. However, serious disadvantages were also found in these systems, since the mesoporous outer walls exhibit a very low hydrothermal stability. Furthermore, it is not possible to effectively control the release of the active principle, since continuous emission through the mesoporous cavities takes place. In order to alleviate this phenomenon, studies have been performed where the mesoporous walls are functionalised with other organic groups that limit and control the exit of the active molecules [Y. Zhu, J. Shi, W. Shen, X. Dong, J. Feng, M. Ruan, Y. Li, Angewandte Chemie International Edition 44 (2005) 5083-5087]. However, the results reported in the literature are not satisfactory.

In order to overcome all the problems listed thus far in systems for the storage and controlled release of active principles, a new type of material is proposed, formed by hybrid nanospheres, which is claimed in this application. More specifically, these spheres contain inside them one or more active principles isolated by a purely organic micellar phase, such as a liposomal phase, and, coating it, an organic-inorganic network is self-assembled, composed, for example, of alternating siliceous units and organic groups. This organo-siliceous coating will make it possible to largely stabilise the micellar or liposomal phase and the active principles, such as bioactive molecules, by protecting them and isolating them from the exterior. Moreover, the inorganic portion of the material will allow for the anchoring of molecules, such as, for example, polyethylene glycol and/or molecular structures designed to increase the selectivity of interaction between the nanoparticles and the desired receptors. Subsequently, when these nanospheres are in the suitable medium and in the presence of a specific external agent, the organic units in the outer coating will break down, thereby allowing for the controlled emission of the active molecules present inside them.

### DESCRIPTION OF THE INVENTION

This invention relates to a hybrid material composed of organic-inorganic particles, characterised in that said particles have a diameter of between 10 and 800 nm, and which is structured in two portions:
- an inner portion that comprises a micellar phase wherein one or more active principles are immersed,
- an outer portion composed of an organic-inorganic network formed by inorganic units and organic units covalently bound to one another, forming a spherical network that coats the micellar phase.

The material of this invention has a variable morphology, and is preferably spherical, the size being within the nanometric range, with diameters ranging between 10 and 800 nm, and, preferably, with a narrow particle size distribution.

In accordance with an additional particular embodiment, said particles are spheres the outer portion whereof is formed by inorganic fragments and organic units composed of an organic group susceptible to being chemically, thermally, enzymatically or photochemically degraded, which causes the breakdown of the organic-inorganic network.

In accordance with an additional particular embodiment, the interior of these nanoparticles is composed of an organic micelle, preferably formed by a phospholipid bilayer with liposomal characteristics which lodges the stabilised active principle or principles therein. A spherical organic-inorganic network, such as an organo-siliceous network composed of covalently bound organic and inorganic units, is self-assembled around said organic micelle.

Preferably, said particles are spheres the inner liposomal portion whereof contains molecules useful in medicine as the active principles.

Said active principles may preferably be any molecule susceptible to being used for therapeutic purposes, such as, for example:
a) analgesic agents,
b) anti-cancer agents,
c) DNA fragments,
d) RNA fragments,
e) biological markers and
f) combinations of two or more of a), b), c), d) and e).

Some specific active principles are, for example, without being limited thereto, ibuprofen, camptothecin and cyclophosphamide.

In accordance with specific embodiments, the nanoparticles of the invention are spheres the outer portion whereof is formed by inorganic fragments and organic units composed of an organic group susceptible to being chemically, thermally, enzymatically or photochemically degraded, which causes the breakdown of the organic-inorganic network.

Preferably, said organic-inorganic network in the outer portion comprises organo-siliceous compounds.

In accordance with a particular embodiment, the structure of the nanoparticles may be divided into two portions: the inner portion and the outer or superficial portion:
(i) Inner portion: Micellar phase with a spherical morphology, formed by an organic bilayer of lipid- or phospholipid-type molecules. Interacting with this medium, there are stabilised active principles, such as bioactive molecules, which are immersed in the innermost aqueous phase, or in the organic bilayer that makes up the most superficial portion of the micellar phase, such as a liposome, depending on the hydrophilic or hydrophobic nature thereof.
(ii) Outer portion: Surrounding said micellar phase, such as a liposome, an organic-inorganic network is organised which is composed of inorganic units, for example, SiO₂, bound to organic groups (R) that may be degraded in response to a stimulus in the medium wherein the molecules or molecular structures, such as, for example, DNA, are to be released. Said hybrid network has successive fractions of the type O_{1.5}Si-R-SiO_{1.5}.

In accordance with an additional particular embodiment, said particles are spheres the outer portion whereof is formed by SiO₂ fragments and organic units selected from acetals, carbamates, esters and disulfides, and, in general, any organic group susceptible to being degraded.

In accordance with particular embodiments, the hybrid material of the invention has a composition that may be expressed by the following empirical formula:

**SiO₂: *w*R : *x*LIP : *y*B : *z*H₂O**

where
***w*** has a value equal to or lower than 0.5, preferably lower than 0.4 and, more preferably, lower than 0.3;
***x*** has a value equal to or lower than 1, preferably lower than 0.5 and, more preferably, lower than 0.2;
***y*** has a value equal to or lower than 1, preferably lower than 0.5 and, more preferably, lower than 0.1;
***z*** has a value equal to or lower than 200, preferably lower than 100 and, more preferably, lower than 50;
**R** is an organic fragment that is introduced between the inorganic units in the outer portion of the nanosphere and which, subsequently, will be degraded as a function of the medium wherein it is located;
**LIP** is the micellar phase, such as a liposomal phase that makes up the inner portion of the nanosphere;
**B** is the active principle that is lodged in the inner liposomal portion and which will exit to the exterior once the organic fragments of the outer portion are degraded.

In accordance with a more preferred embodiment, in said formula,
***w*** has a value equal to or lower than 0.3;
***x*** has a value equal to or lower than 0.2;
***y*** has a value equal to or lower than 0.1;
***z*** has a value equal to or lower than 50;
**R** is an organic fragment that is introduced between the inorganic units in the outer portion of the nanosphere and which, subsequently, will be degraded as a function of the medium wherein it is located;
**LIP** is the liposomal phase that makes up the inner portion of the nanosphere;
**B** is the active principle that is lodged in the inner liposomal portion and which will exit to the exterior once the organic fragments of the outer portion are degraded.

In an even more preferred embodiment, the hybrid material defined above has a composition such that, in said empirical formula:
***w*** has a value equal to or lower than 0.3;
***x*** has a value equal to or lower than 0.2;
***y*** has a value equal to or lower than 0.1;
***z*** has a value equal to or lower than 50;
   - said particles are spheres the outer portion whereof is formed by SiO₂ fragments and organic units selected from acetals, carbamates, esters and disulfides, and the inner liposomal portion whereof contains active principles selected from:
      a) analgesic agents,
      b) anti-cancer agents,
      c) DNA fragments,
      d) RNA fragments,
      e) biological markers and
      f) combinations of two or more of a), b), c), d) and e).

The nanoparticulate system disclosed herein has a thermal stability typical of organo-siliceous materials, which ranges between 300ºC and 600ºC. The organic groups present in the outer portion are susceptible to being decomposed as a function of the medium wherein they are located, through different reactions, such as, for example, acid or base hydrolysis, oxidation, reduction, thermal, photochemical or enzymatic reactions. These processes favour the breakdown of the outer organic-inorganic portion, allowing for the release of the active principles that are immersed in the inner liposomal phase.

This invention also relates to a method of preparing organic-inorganic hybrid nanoparticles which are capable of storing and releasing, in a controlled manner, active principles stabilised therein.

Thus, a second object of the invention is a method of synthesising the material composed of nanoparticles defined above, which comprises two steps.
- a first step wherein an aqueous micellar phase is prepared, such as a liposomal phase, where one or more active principles, such as bioactive molecules, are encapsulated, which comprises forming a first emulsion of said active principles dissolved in an organic solvent with water, forming a second emulsion,
- a second step wherein a spherical organic-inorganic network is formed around the micellar phase, such as liposomes, loaded with active principles, prepared in the preceding step.

In accordance with a particular embodiment, the first step comprises dissolving lipid or phospholipid molecules in an organic solvent.

The concentration of said lipid or phospholipid molecules in the organic phase ranges between 0.05 mM and 20 mM, preferably 1.00 mM.

Said phospholipid is preferably lecithin.

Said organic solvent is preferably chloroform.

In accordance with a particular embodiment, the first step comprises dissolving molecules selected from lipids and phospholipids in chloroform, the concentration of said lipids and phospholipids in the organic phase being 1.00 mM. In particular, said first step comprises:
- forming a first emulsion using water
- adding de-ionised water,
- forming a second emulsion, which is kept under stirring for a period of time of between 30 minutes and 48 hours, to form an aqueous suspension of liposomes that contain the active principle and
- subjecting the suspension to centrifugation.

In an additional particular embodiment of the method, the first step comprises dissolving molecules selected from lipids and phospholipids in chloroform, the concentration of said lipid or phospholipid molecules in chloroform being 1.00 mM, and forming a first emulsion using water, adding de-ionised water, forming a second emulsion, which is kept under stirring, to form an aqueous suspension of liposomes that contain one or more active principles, and subjecting the suspension to centrifugation until a concentration of 100 mg of liposome per 1 ml of de-ionised water is obtained.

In accordance with a preferred embodiment, in the first step, an aqueous liposomal phase is prepared wherein one or more active principles, such as water-soluble or insoluble bioactive molecules, are encapsulated. To this end, lipid or phospholipid molecules (preferably lecithin) are dissolved in an organic solvent (preferably chloroform) wherein one or more active principles have been previously dissolved. The concentration of lipids in the organic phase ranges between 0.05 mM and 20 mM, preferably 1.00 mM. The quantity of water used ranges between 1 and 100 ml, preferably 10 ml and, more preferably, 5 ml. The mixture formed is emulsified by stirring between 1,000 and 10,000 rpm, preferably 5,000 rpm, for 1 to 120 minutes, preferably 10 minutes, for a suitable time and under adequate conditions to eliminate the organic solvent incorporated at the beginning, to form a first emulsion. Subsequently, between 50 and 1,000 ml of de-ionised water, preferably 200 ml, are added, and it is kept at between 35ºC and 100ºC, preferably 45ºC, under constant stirring, for 60 minutes to 24 hours, preferably 120 minutes. Once this period has elapsed, a second emulsion is formed, which is kept under stirring for a time between 30 minutes and 48 hours, preferably 120 minutes, to form an aqueous suspension of liposomes that contain the active principle. Finally, by subjecting the solution to a centrifugation process at 15,000 rpm for a time that should never be less than 120 minutes, a concentration of 100 mg of liposome per 10 ml of de-ionised water, more preferably of 100 mg of liposome per 1 ml of de-ionised water, is obtained. Finally, the liposomal phase is separated from the aqueous phase, to reach the desired concentration of liposome in water.

The active principles are dissolved either in the organic solvent or the aqueous solvent, depending on the hydrophilic properties thereof.

In accordance with a particular embodiment of the method, the second step comprises:
- forming the spherical organic-inorganic network around a micellar phase, preferably formed by liposomes, loaded with one or more active principles, such as bioactive molecules, prepared in the first step, by means of the following steps:
- performing hydrolysis and condensation between organo-siliceous precursors,
- preparing a reaction mixture formed by disilane and/or monosilane molecules, the aqueous suspension of the micellar phase and de-ionised water,
- keeping the reaction mixture under constant stirring, between 200 and 500 rpm, at ambient temperature for a period of between 12 and 96 hours,
- adding an inorganic salt, in a proportion that represents between 1% and 15% of the total silicon incorporated into the reaction mixture,
- keeping it under constant stirring for a period of between 12 and 170 hours and,
- recovering the nanospheres by centrifugation.

In a more specific embodiment of the method, the second step comprises:
- forming the spherical organic-inorganic network around the liposomes loaded with one or more active principles, such as bioactive molecules, prepared in the first step, by means of the following steps:
- performing hydrolysis and condensation between organo-siliceous precursors, said precursors being disilanes with the molecular formula (R'O)₃Si-R-Si(OR')₃,
- preparing a reaction mixture formed by disilane and/or monosilane molecules, the aqueous suspension of liposomes and de-ionised water, said mixture having the following composition:

   **SiO₂ : *m*LIP : *n*H₂O**

   where
   ***m*** has a value equal to or lower than 1, preferably lower than 0.5 and, more preferably, lower than 0.2;
   ***n*** has a value equal to or lower than 200, preferably lower than 100 and, more preferably, lower than 50;
   **LIP** is the liposomal phase that makes up the inner portion of the nanosphere,
- keeping the reaction mixture under constant stirring, between 200 and 500 rpm, at ambient temperature for a period of between 1 and 96 hours, more preferably between 12 and 96 hours, and, more preferably, between 12 and 80 hours,
- adding an inorganic salt, in a proportion that represents between 1% and 15% of the total silicon incorporated into the reaction mixture, preferably 4% of the total silicon incorporated into the reaction mixture,
- keeping it under constant stirring for a period of between 1 and 170 hours, preferably between 12 and 150 hours, and,
- recovering the nanospheres by centrifugation at between 12,000 and 18,000 rpm, preferably 15,000 rpm, for 2-4 hours, and drying at between 50ºC and 75ºC, preferably 60ºC, for a period of between 10 minutes and 50 hours, preferably between 24 and 48 hours.

In accordance with an additional particular embodiment, the siliceous precusors used in the second step are monosilanes and disilanes, the mole percent of silicon that corresponds to disilane and monosilane ranging between 5 and 100 mole percent of silicon from disilane, and between 95 and 0 mole percent of silicon from monosilane.

In accordance with an additional particular embodiment, in the second step, organic groups selected from acetals, carbamates, esters and disulfides and, in general, any organic group susceptible to being degraded, are inserted in the outer network of the spheres.

In the second step, the final hydrolysis and condensation between disilane molecules may be performed in the presence of monosilanes, such as monosilanes selected from Aerosil, Ludox and tetraethyl orthosilicate (TEOS).

The inorganic salt may be, for example, an ammonium or sodium halide, such as ammonium fluoride or sodium fluoride.

The disilanes for the second step may be prepared in a previous step following the methodology widely described in the literature, which basically consists of: i) reactions with organo-magnesium-type reagents, following the Grignard methodology, or organo-lithium-type reagents, which react with siliceous compounds, ii) sililations of aryl bromides or iodides using palladium catalysts, iii) condensations using suitable triethoxysilanes.

Using these disilanes, it will be possible to insert different organic groups, R, such as, for example, acetals, carbamates, esters, disulfides and, in general, any organic group susceptible to being degraded, causing the breakdown of the organic-inorganic network and releasing the active principle. The presence in these disilanes of, for example, terminal alkoxy groups, OR', where R' is an alkoxy radical with a variable number of carbon atoms, preferably between 1 and 10 carbon atoms, of the methoxide, ethoxide or propoxide type, will facilitate the subsequent condensation between them to form the organic-inorganic network.

The final hydrolysis and condensation between, for example, disilane molecules, may be performed in the presence or absence of monosilanes, such as, for example, Aerosil, Ludox, tetraethyl orthosilicate (TEOS) or any other known source of silicon.

During the entire second step of the method, it is convenient to keep the reaction medium in total darkness, in order to preserve the integrity of the liposomal phase loaded with the active principle, thereby preventing the decomposition thereof.

The nanoparticles, preferably spheres, of nanometric size finally obtained, wherein bioactive molecules are immersed in a liposomal phase, which, in turn, is coated with an organic-inorganic network, are susceptible to being used as storage and emission systems for active principles. Thus, for example, they could be used for the emission of active principles in living beings. In order to prove this statement, it is necessary to subject said nanospheres *in vitro* to the actual conditions wherein they are to act. These conditions will be specific to degrade the organic groups present in the outer portion of the spheres, thereby allowing for the selective, controlled emission of the active principles inside them. In this way, we will be able to incorporate a highly specific organic group into the outer network that is susceptible to being decomposed, as a function of the place and the conditions of the medium wherein the hybrid nanospheres are to be located. The examples shown below illustrate some *in vitro* cases which prove the emission of bioactive molecules.

The organic-inorganic hybrid materials of the invention are useful for the storage and subsequent controlled release of molecules, especially in the field of medicine. The breakdown of the organic fragments present in the outer portion of these nanoparticulate materials, by the action of external chemical agents present in the medium, allows for the controlled release of molecules present inside the particles, through the "open doors" generated on the outer surface.

### BRIEF DESCRIPTION OF THE FIGURES

The following figures are included as an integral part of this specification:

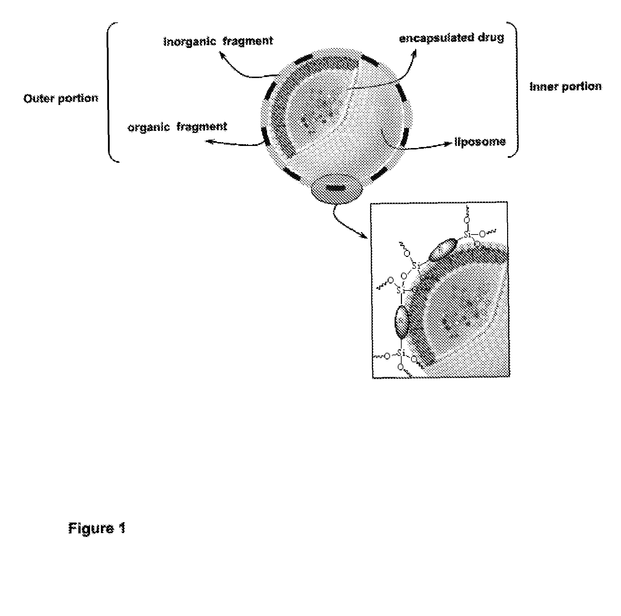

Figure 1 is a graphic representation of the nanospheres described herein, which indicates the different portions that make them up.

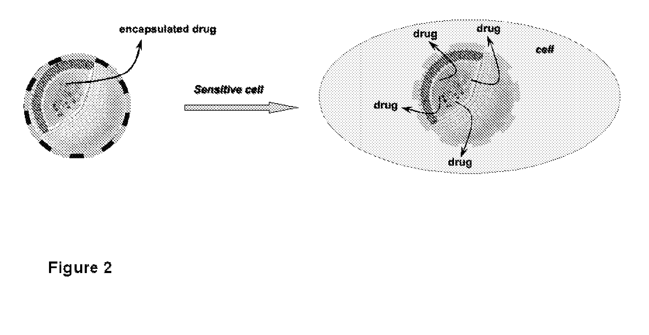

Figure 2 shows a diagram of the release process of the active molecules which takes place following the breakdown of the organic fragments located in the outer portion of the nanosphere; it illustrates the exit process of the bioactive molecules following the breakdown of the outer network.
Figure 3 is a ¹³C NMR spectrum of a disilane with the molecular formula (R'O)₃Si-R-Si(OR')₃, prepared to coat the liposomal phase, which contains acetal groups (R).
Figure 4 is a ¹³C NMR spectrum of the material composed of nanospheres, the outer portion whereof contains intact acetal groups.
Figure 5 is a ²⁹Si NMR spectrum of the material composed of nanospheres, which demonstrates that the outer portion is formed by organic and inorganic units linked to one another.
Figure 6 shows transmission electron microscopy photographs of the material obtained, where spheres of nanometric size may be observed.
Figure 7 shows a graph that represents the percentage of ibuprofen molecules that exit to the exterior after treating the nanospheres at pH=4 for different periods of time.
Figure 8 is a ¹³C NMR spectrum of the material composed of nanospheres following treatment at pH=4 for 15 minutes, where it may be observed that the acetal groups present in the outer network have been hydrolysed.
Figure 9 shows transmission electron microscopy photographs of the sample subjected to pH=4 for a period of 15 minutes, where nanometric spheres are no longer observed.
Figure 10 shows a graph that represents the percentage of ibuprofen molecules that exit to the exterior after treating the nanospheres at pH=7.5 for different periods of time.

### EMBODIMENTS OF THE INVENTION

The following embodiment examples of the invention are shown below.

### Example 1: Preparation of a liposomal phase containing ibuprofen

This first example describes the preparation of spherical liposomes in an aqueous phase, which contain molecules of active principles inside them, specifically ibuprofen. To this end, 1.0 g of lecithin (phosphatidylcholine) is dissolved in 10 ml of chloroform wherein 0.128 g of ibuprofen had been dissolved. Once a transparent suspension is formed, 5 ml of de-ionised water are added and the suspension is subjected to strong stirring at 5,000 rpm for 10 minutes. After this period has elapsed, a first emulsion is formed.

Subsequently, 150 ml of de-ionised water are added, slowly and under constant stirring, to form a suspension that is kept at 45ºC for 120 minutes, in order to achieve the elimination, by evaporation, of the chloroform initially incorporated. Once this time has elapsed, a second emulsion is formed, which is kept under stirring for 120 minutes, to generate an aqueous suspension of liposomes.

Finally, said suspension is subjected to a centrifugation process at 15,000 rpm for 120 minutes, in order to precipitate a concentrated liposome phase, eliminating most of the water used in the process. The concentration finally obtained in the liposomal phase was 100 mg of liposome per 1 ml of de-ionised water.

Therefore, by following this method, liposomes in the aqueous phase have been obtained which are formed by a lecithin bilayer and contain ibuprofen inside them. Ultraviolet-visible spectroscopy studies performed on the aqueous phase that is separated from the liposomal phase show the absence of ibuprofen, which allows us to conclude that all the active principle used in the preparation has been introduced into the liposomal phase.

In ultraviolet-visible spectroscopy, the ibuprofen molecules are monitored by observing the band centred at 264 nm, which is characteristic of this active principle.

### Example 2: Preparation of an organo-siliceous precursor of the type (R'O)₃Si-R-Si(OR')₃, where R is an acetal group.

A mixture formed by benzaldehyde (10 mmol), triethyl orthoformate (10 mmol), anhydrous methanol (0.02 mmol) and hydroxymethyltriethoxysilane (30 mmol) in dichloromethane (50 ml) is prepared. Once it is homogenised, NBS (5% mol) is added to this initial mixture. The resulting solution is stirred at ambient temperature until the aldehyde is completely consumed. Following this process, the solvent is eliminated and a reaction crude remains which is purified by vacuum distillation and which has a yield of 32%. The product formed (bis-(triethoxysilane-methoxy-methyl))-benzene, has the following molecular formula:

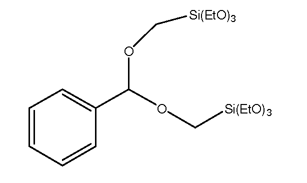

The NMR results of this product are listed below: ¹H NMR (400 MHz, CDCl₃): δ = 1.23 (18H, t, *J* = 6.9Hz, *CH*₃CH₂); 3.66 (4H, s, *CH₂*O); 3.87 (12H, q, *J* = 6.3Hz, CH₃C*H*₂); 5.51 (1H, s, C*H*O); 7.24-7.50 (5H, m, AROM); ¹³C NMR (100.6 MHz, CDCl₃): δ = 18.2, 58.7, 61.2, 102.8, 126.8, 127.9, 138.4.

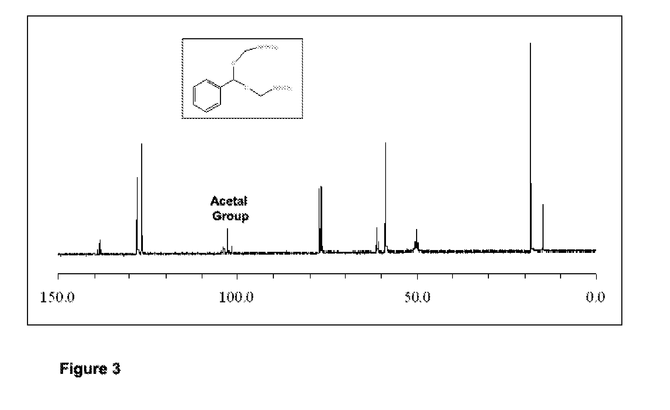

Figure 3 shows the ¹³C NMR spectrum of the organo-siliceous precursor described herein.

### Example 3: Preparation of the organic-inorganic network around the liposomes loaded with ibuprofen.

This example describes the final preparation step for the hybrid spheres formed by an outer organo-siliceous network, which has acetal groups susceptible to being hydrolysed, and an inner portion formed by a liposomal phase that contains ibuprofen molecules.

To this end, 15.750 ml of the liposomal phase containing ibuprofen, in a concentration of 100 mg per 1 ml of de-ionised water (prepared in example 1), are added to a mixture, previously formed, of 1.734 g of TEOS and 1.989 g of disilane-acetal (prepared in example 2). The suspension formed is kept under stirring (300 rpm) for 24 hours; thereafter, 0.028 g of NaF are added in order to initiate the condensation process of the silane groups. The stirring process is maintained for another 48 hours and, subsequently, the solid formed is recovered by centrifugation at 15,000 rpm for 2 hours in the presence of distilled water. Finally, the solid formed is oven-dried at 60ºC for 24 hours.

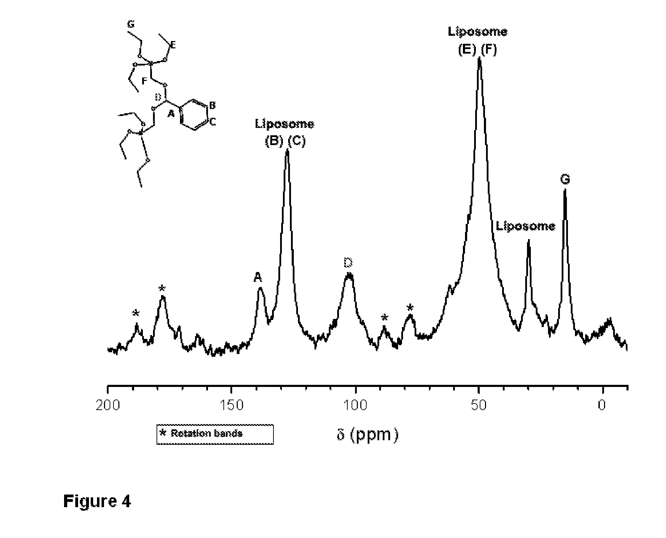

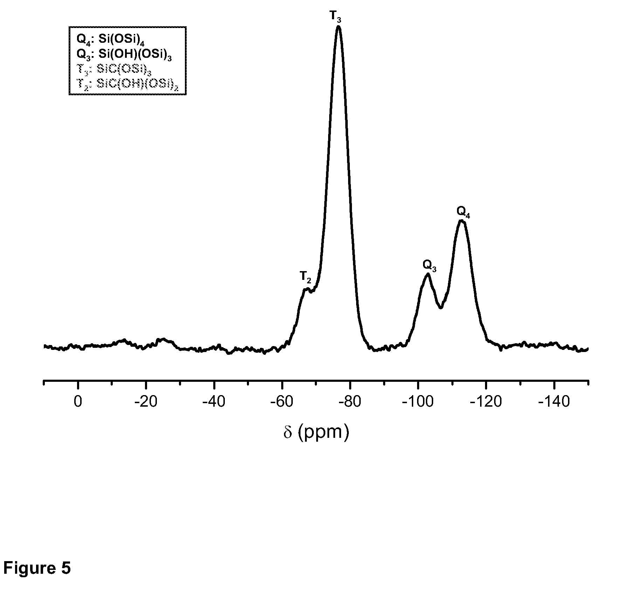

Figure 4 and Figure 5 show the ¹³C and ²⁹Si NMR spectra obtained for the sample prepared, respectively. In the case of the ¹³C NMR spectrum, all the bands assigned to the carbon atoms present in the starting disilane are observed, including the acetal group located at ∼100 ppm, which shows the integrity of the organo-siliceous network. On the other hand, the ²⁹Si NMR spectrum shows the bands characteristic of the Si-C bonds located at -62 and -76 ppm, corresponding to silicon atoms of types T₂ (SiC(OH)(OSi)₂) and T₃ (SiC(OSi)₃), jointly with the bands characteristic of polymerised silica of types Q₃ (Si(OH)(OSi)₃) and Q₄ (Si(OSi)₄) located at -102 pmm and -112 ppm, respectively. These results show that the outer organic-inorganic network that envelops the liposomes has been formed, covalently linking acetal-type organic groups and SiO₂ in an alternating manner.

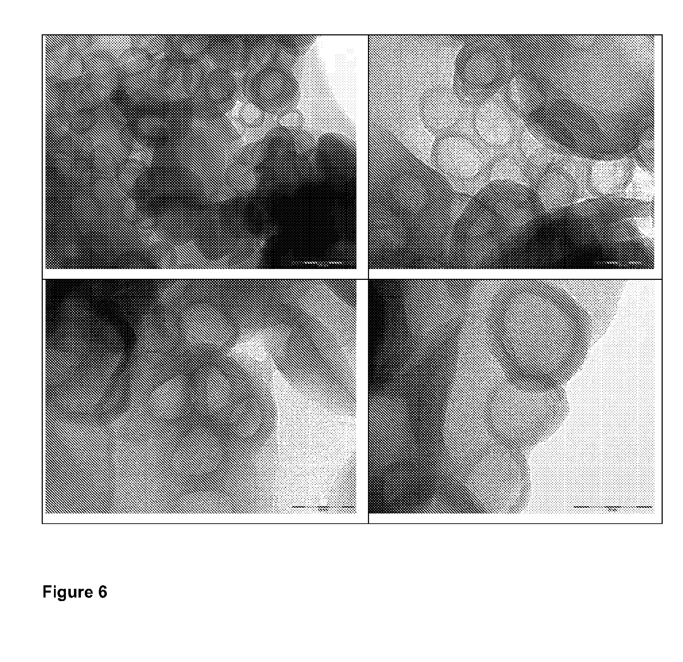

Figure 6 shows different transmission electron microscopy images, where the nanospheres formed, which have diameters of between 50 and 150 nm, may be observed.

### Example 4: Breakdown process of the organic group in the outer network and release of ibuprofen using a medium at pH=4.

In this example, the acetal groups present in the outer network of the nanospheres obtained in example 3 are hydrolysed, with the consequent release of the ibuprofen molecules that were stored in the inner liposomal portion.

To this end, the sample obtained in example 3 is divided into different vials; 0.020 g of the sample composed of nanospheres are introduced into each of them, forming a suspension with 10 ml of a solution of HCl/EtOH, such that the pH remains constant at a value of 4.0. In each vial, the maximum ibuprofen concentration that could be released is 1.4 x 10⁻³ M. The suspensions in each vial are kept under constant stirring (300 rpm) for different times (5, 15, 30, 45, 60, 90 and 120 minutes); thereafter, the stirring is stopped and the sample is filtered; the filtered liquid is recovered and analysed by means of ultraviolet-visible spectroscopy. The ibuprofen concentration present in the recovered liquid was monitored using this technique, by measuring the absorbance of the band located at 264 nm, taking into consideration that the calibration curve for this compound was A = (181.32 x C) - 0.0007, where A is the absorbance of this band and C is the ibuprofen concentration.

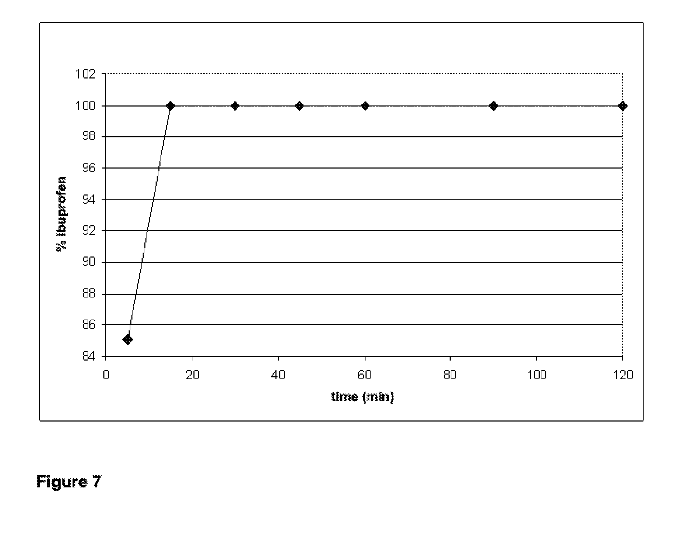

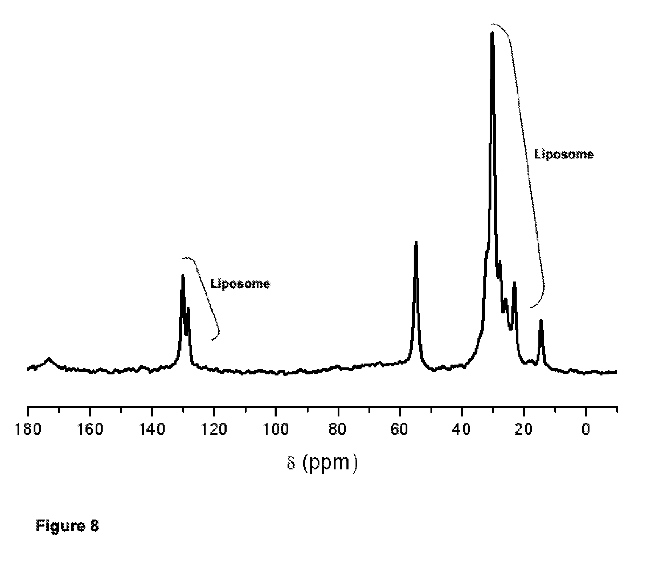

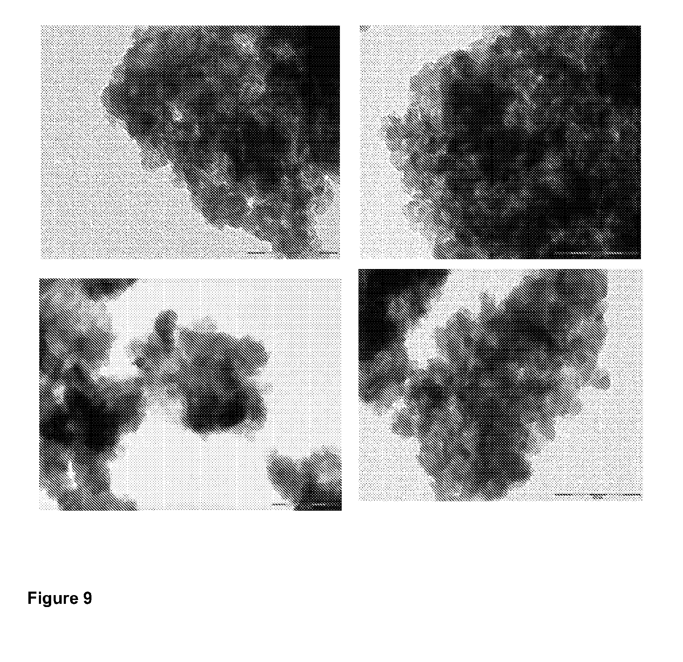

The results obtained are shown in Figure 7; they demonstrate that, at pH=4, the acetal groups are hydrolysed, causing the ibuprofen molecules to exit to the exterior. After 15 minutes, all the ibuprofen has been released. Figure 8 shows the ¹³C NMR spectrum of the sample subjected to 15 minutes of treatment with the solution at pH=4; it may be observed that the band located at ∼100 ppm, characteristic of the acetalic groups, has disappeared. This fact confirms the hydrolysis of these organic groups and, therefore, the breakdown of the outer network that coated the liposomal phase. Figure 9 shows the transmission electron photographs for the same sample treated at pH=4, where the presence of nanospheres is no longer observed, since they have been degraded, leading to the release of the active principles stored therein.

### Example 5: Breakdown process of the organic group in the outer network and release of ibuprofen using a medium at pH=7.5.

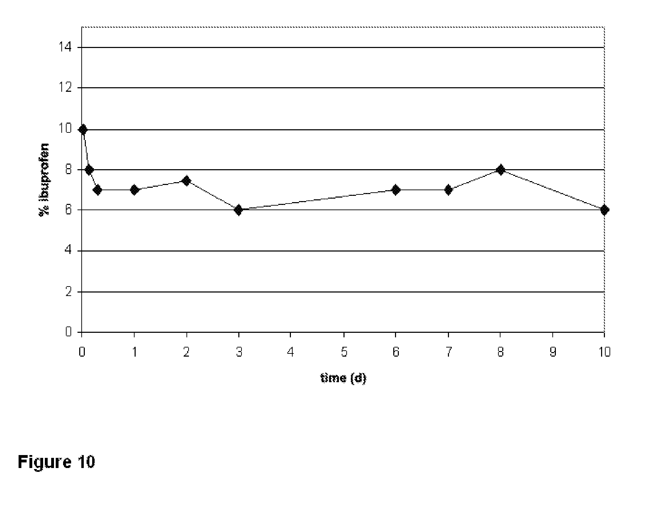

The experiment performed in example 4 is repeated, this time subjecting the ibuprofen-containing nanospheres to a treatment with a solution in EtOH at pH=7.5. The experiments were performed at 30 minutes, 3 and 7 hours, and, 1, 2, 3, 6, 7, 8 and 10 days. Figure 10 shows the results obtained in regards to the exit of ibuprofen; it may be observed that the presence of the active principle remains constant, between 8% and 10%; it must be residual ibuprofen present on the outer surface of the nanospheres, since no exit of the internal drug was detected, as was the case in example 4. The ¹³C NMR spectrum of the sample treated for 7 days shows the band located at ∼100 ppm characteristic of the acetal-type organic groups, which confirms that, under these pH=7.5 conditions, the hydrolysis and breakdown of the outer hybrid network does not take place.

## Claims

1. A hybrid material composed of organic-inorganic particles, **characterised in that** said particles have a diameter of between 10 and 800 nm, and it is structured in two portions:
- an inner portion that comprises a micellar phase wherein one or more active principles are immersed,
- an outer portion composed of an organic-inorganic network formed by inorganic units and organic units covalently bound to one another, forming a spherical network that coats the micellar phase.

2. A composite material as claimed in claim 1, **characterised in that** said micellar phase is of a liposomal nature, selected from a lipid-type phase and a phospholipid-type phase.

3. A composite material as claimed in claim 1, **characterised in that** said particles are spheres the inner micellar portion whereof contains molecules useful in medicine as the active principles.

4. A composite material as claimed in claim 3, **characterised in that** said active principles are selected from:
a) analgesic agents,
b) anti-cancer agents,
c) DNA fragments,
d) RNA fragments,
e) biological markers and
f) combinations of two or more of a), b), c), d) and e).

5. A composite material as claimed in claim 3, **characterised in that** said active principles are selected from ibuprofen, camptothecin and cyclophosphamide.

6. A composite material as claimed in claim 1, **characterised in that** said particles are spheres the outer portion whereof is formed by inorganic fragments and organic units composed of an organic group susceptible to being chemically, thermally, enzymatically or photochemically degraded, which causes the breakdown of the organic-inorganic network.

7. A composite material as claimed in claim 1, **characterised in that** said organic-inorganic network in the outer portion comprises organo-siliceous compounds.

8. A composite material as claimed in claim 6, **characterised in that** said particles are spheres the outer portion whereof is formed by SiO₂ fragments and organic units selected from acetals, carbamates, esters and disulfides.

9. A composite material as claimed in any of the preceding claims, **characterised in that** its composition may be expressed by the following empirical formula:
**SiO₂: *w*R : *x*LIP: *y*B : *z*H₂O**
where
***w*** has a value equal to or lower than 0.5;
***x*** has a value equal to or lower than 1;
***y*** has a value equal to or lower than 1;
***z*** has a value equal to or lower than 200;
**R** is an organic fragment that is introduced between the inorganic units in the outer portion of the nanosphere, which is susceptible to being degraded;
**LIP** is the liposomal phase that makes up the inner portion of the nanosphere;
**B** is the active principle that is lodged in the inner liposomal portion and which will exit to the exterior once the organic fragments of the outer portion are degraded.

10. A composite material as claimed in claim 9, **characterised in that**, in said formula,
***w*** has a value equal to or lower than 0.3;
***x*** has a value equal to or lower than 0.2;
***y*** has a value equal to or lower than 0.1;
***z*** has a value equal to or lower than 50;
**R** is an organic fragment that is introduced between the inorganic units in the outer portion of the nanosphere, and which is susceptible to being degraded;
**LIP** is the liposomal phase that makes up the inner portion of the nanosphere;
**B** is the active principle that is lodged in the inner liposomal portion and which will exit to the exterior once the organic fragments of the outer portion are degraded.

11. A composite material as claimed in claim 9, **characterised in that**, in said empirical formula:
***w*** has a value equal to or lower than 0.3;
***x*** has a value equal to or lower than 0.2;
***y*** has a value equal to or lower than 0.1;
***z*** has a value equal to or lower than 50;
- said particles are spheres the outer portion whereof is formed by SiO₂ fragments and organic units selected from acetals, carbamates, esters and disulfides, and the inner liposomal portion whereof contains one or more active principles selected from:
a) analgesic agents,
b) anti-cancer agents,
c) DNA fragments,
d) RNA fragments,
e) biological markers and
f) combinations of two or more of a), b), c), d) and e).

12. A method of synthesising the material composed of nanoparticles defined in any of the claims, **characterised in that** it comprises two steps:
- a first step where an aqueous micellar phase is prepared wherein one or more active principles are encapsulated, which comprises forming a first emulsion of said active principles dissolved in an organic solvent with water and forming a second emulsion,
- a second step wherein a spherical organic-inorganic network is formed around the micellar phase, loaded with active principles, prepared in the preceding step.

13. A method as claimed in claim 12, **characterised in that** the first step comprises dissolving lipid or phospholipid molecules in an organic solvent.

14. A method as claimed in claim 13, **characterised in that** the concentration of said lipid or phospholipid molecules in the organic phase ranges between 0.05 mM and 20 mM, preferably 1.00 mM.

15. A method as claimed in any of claims 12 to 14, **characterised in that** said phospholipid is lecithin.

16. A method as claimed in claim 12, **characterised in that** said organic solvent is chloroform.

17. A method as claimed in claim 12, **characterised in that** the first step comprises dissolving molecules selected from lipids and phospholipids in chloroform, the concentration of said lipids and phospholipids in the organic phase being 1.00 mM.

18. A method as claimed in claim 12, **characterised in that** said first step comprises:
- forming a first emulsion using water,
- adding de-ionised water,
- forming a second emulsion, which is kept under stirring for a time of between 30 minutes and 48 hours, to form an aqueous suspension of the micellar phase, preferably liposomes that contain the active principle, and
- subjecting the suspension to centrifugation.

19. A method as claimed in claim 12, **characterised in that** the first step comprises dissolving molecules selected from lipids and phospholipids in chloroform, wherein one or more active principles have been previously dissolved, the concentration of said lipid or phospholipid molecules in chloroform being 1.00 mM, and forming a first emulsion using water, adding de-ionised water, forming a second emulsion, which is kept under stirring, to form an aqueous suspension of liposomes that contain one or more active principles, and subjecting the suspension to centrifugation until a concentration of 100 mg of liposome per 1 ml of de-ionised water is obtained.

20. A method as claimed in claim 12, **characterised in that** the second step comprises:
- forming the spherical organic-inorganic network around a micellar phase loaded with one or more active principles, prepared in the first step, by means of the following steps:
- performing hydrolysis and condensation between organo-siliceous precursors,
- preparing a reaction mixture formed by disilane and/or monosilane molecules, the aqueous suspension of the micellar phase and de-ionised water,
- keeping the reaction mixture under constant stirring, between 200 and 500 rpm, at ambient temperature for a period of between 12 and 96 hours,
- adding an inorganic salt, in a proportion that represents between 1% and un 15% of the total silicon incorporated into the reaction mixture,
- keeping it under constant stirring for a period of between 12 and 170 hours, and,
- recovering the nanospheres by centrifugation.

21. A method as claimed in claim 19, **characterised in that** the second step comprises:
- forming the spherical organic-inorganic network around the liposomes, loaded with bioactive molecules, prepared in the first step, by means of the following steps:
- performing hydrolysis and condensation between organo-siliceous precursors, said precursors being disilanes with the molecular formula (R'O)₃Si-R-Si(OR')₃,
- preparing a reaction mixture formed by disilane and/or monosilane molecules, the aqueous suspension of liposomes and de-ionised water, said mixture having the following composition:
**SiO₂ : *m*LIP : *n*H₂O**
where
***m*** has a value equal to or lower than 1;
***n*** has a value equal to or lower than 200;
**LIP** is the liposomal phase that makes up the inner portion of the nanosphere,
- keeping the reaction mixture under constant stirring, between 200 and 500 rpm, at ambient temperature for a period of between 12 and 96 hours,
- adding an inorganic salt, in a proportion that represents 4% of the total silicon incorporated into the reaction mixture,
- keeping it under constant stirring for a period of between 12 and 170 hours, and,
- recovering the nanospheres by centrifugation at 15,000 rpm for 2-4 hours and drying at 60ºC for a period of between 24 and 48 hours.

22. A method as claimed in claims 12 or 20, **characterised in that**, in the second step, siliceous precursors selected from disilanes, monosilanes and combinations of both are used, the mole percent of silicon that corresponds to disilane and monosilane ranging between 5 and 100 mole percent of silicon from disilane, and between 95 and 0 mole percent of silicon from monosilane.

23. A method as claimed in claim 12, **characterised in that**, in the second step, organic groups selected from acetals, carbamates, esters and disulfides are inserted in the outer network of the spheres.

24. A method as claimed in claim 20, **characterised in that**, in the second step, the final hydrolysis and condensation between disilane molecules is performed in the presence of monosilanes selected from Aerosil, Ludox and tetraethyl orthosilicate (TEOS).

25. A method as claimed in claim 20, **characterised in that**, in the second step, the inorganic salt is ammonium fluoride or sodium fluoride.

26. Use of a composite material defined in any of claims 1 to 11, for the storage inside it and the release of active principles.

27. Use of a composite material as claimed in claim 26, for the storage and subsequent controlled release of active principles to be used in medicine.
